# EUROPEAN PATENT SPECIFICATION

(11) **EP 3 334 397 B1**
(45) Date of publication and mention of the grant of the patent: **17.03.2021**
(21) Application number: 16754094.7
(22) Date of filing: 11.08.2016
(51) Int. Cl.: A61F 13/42, A61F 13/496, A61F 13/514, A61F 13/513, A61F 13/64, A61F 13/84, A61F 13/551, A61F 13/00

(54) **BELTED STRUCTURE WITH GRAPHICS**
GEGURTETE STRUKTUR MIT GRAFIK
STRUCTURE À CEINTURE COMPRENANT DES ÉLÉMENTS GRAPHIQUES

(30) Priority: 13.08.2015 US 201562204680 P; 16.05.2016 US 201662336775 P
(43) Date of publication of application: 20.06.2018
(73) Proprietor: The Procter & Gamble Company, Cincinnati, OH 45202 (US)
(72) Inventor: LAVON, Gary Dean, Cincinnati, Ohio 45202 (US)
(74) Representative: P&G Patent Germany
(86) International application number: PCT/US2016/046510
(87) International publication number: WO 2017/027683

(56) References cited:
- EP-A1- 2 258 329
- WO-A1-2013/159273
- WO-A1-2013/170433
- US-A1- 2010 274 213
- US-A1- 2015 088 088

## Description

### FIELD

The present disclosure generally relates to pull-on disposable absorbent articles comprising an elastomeric belt, a central chassis, and graphics.

### BACKGROUND

A particular type of absorbent article pant design currently marketed is sometimes called the "balloon" pant. The balloon pant design usually includes a central absorbent chassis and an elastic belt. The elastic belt is usually relatively wide (in the longitudinal direction) and elastically stretchable in the lateral direction. It entirely encircles the wearer's waist, and thereby covers a relatively large amount of the wearer's skin, and also makes up a relatively large portion of the visible outside surfaces of the pant. The central chassis portion is typically joined to the inside of the belt in the front, wraps under the wearer's lower torso between the legs, and is joined to the inside of the belt in the rear. As such, balloon pants are a compilation of separate article components. Because of the manner in which absorbent article components are incorporated, even the viewable surfaces of the article may have seams or areas of overlap or connection. WO2013/170,433A1 and WO2013/159,273A1 disclose belted absorbent articles with graphics on the chassis or on the belt.

Thus, it is challenging to create an absorbent article that comprises graphics on multiple absorbent article components such that the graphics flow from absorbent article component to component in a manner that deemphasizes seams and creates a holistic underwear-like appearance. It is also challenging to place graphics on these individual components and to line them up such that the multi-component construction appears to be an integrated structure wherein the seams are de-emphasized and process variations are masked. Therefore, any improvement in the placement of graphics on a belted article with components can enhance its appearance and functionality and may give the manufacturer an advantage.

### BRIEF DESCRIPTION OF THE DRAWINGS

Fig. 1 is a perspective view of one example of a belted article.
Fig. 2 is a schematic plan view of a belted article precursor structure, prior to joining of the front and rear sections of the belt.
Fig. 3 is a schematic plan view of a belted article precursor structure with a first graphic and various second graphic objects according to the claimed invention.
Fig. 4 is a schematic plan view of a belted article precursor structure with various graphic objects.
Fig. 5 is a partial schematic plan view of a belted article precursor structure with a graphic object.
Fig. 6a is a schematic plan view of a central chassis of a belted article.
Fig. 6b is a schematic plan view of a front belt portion of a belted article.
Fig. 6c is a schematic plan view of the front belt portion of figure 6b combined with the central chassis of figure 6a.
Fig. 7 is schematic side view of a belted article with various graphic objects.
Fig. 8 is a top schematic view of the belted article of figure 7.
Fig. 9a is a top schematic view of a belted article.
Fig. 9b is a schematic side view of the belted article of figure 9a.

### DETAILED DESCRIPTION

Various non-limiting embodiments of the present disclosure will now be described to provide an overall understanding of the principles of the structure, function, manufacture, and use of the absorbent articles disclosed herein. One or more examples of these non-limiting embodiments are illustrated in the accompanying drawings. Those of ordinary skill in the art will understand that the absorbent articles described herein and illustrated in the accompanying drawings are non-limiting example embodiments and that the scope of the various non-limiting embodiments of the present disclosure are defined solely by the claims. The features illustrated or described in connection with one non-limiting embodiment may be combined with the features of other non-limiting embodiments. Such modifications and variations are intended to be included within the scope of the present disclosure.

The following term explanations may be useful in understanding the present disclosure:
"Absorbent article" refers to pull-on garments generally worn by infants and other incontinent individuals to absorb and contain urine, feces and/or menses. It should be understood, however, that the term absorbent article is also applicable to other garments such as training pants, incontinent briefs, feminine hygiene garments or panties, and the like. In some embodiments, "absorbent article" may refer to a taped diaper.

The terms "elastic," "elastomer," and "elastomeric" refer to a material which generally is able to extend to a strain of at least 50% without breaking or rupturing, and is able to recover substantially to its original dimensions after the deforming force has been removed.

As used herein, "graphic" refers to formation of an object, which may or may not be colored. A graphic, however, does not include a field of color alone, wherein no formation of an object exists.

As used herein, "graphic absorbent article component" refers to a component of an absorbent article which comprises a graphic. Graphic absorbent article components may additionally comprise color, including color accents, or a field of color which does not form part of an object.

"Lateral", with respect to a pant and its wearer, refers to the direction generally perpendicular with the wearer's standing height, or the horizontal direction when the wearer is standing. "Lateral" is also the direction generally perpendicular to a line extending from the midpoint of the front waist edge to the midpoint of the rear waist edge.

"Longitudinal", with respect to a pant and its wearer, refers to the direction generally parallel with the wearer's standing height, or the vertical direction when the wearer is standing. "Longitudinal" is also the direction generally parallel to a line extending from the midpoint of the front waist edge to the midpoint of the rear waist edge.

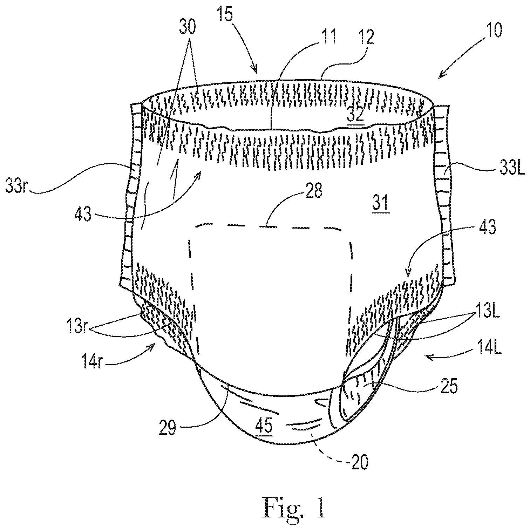

As used herein, the term "pull-on garment" refers to articles of wear which have a defined waist opening and a pair of leg openings and which are pulled onto the body of the wearer by inserting the legs into the leg openings and pulling the article up over the waist. The term "disposable" is used herein to describe garments which are not intended to be laundered or otherwise restored or reused as a garment (i.e., they are intended to be discarded after a single use and, preferably, to be recycled, composted or otherwise disposed of in an environmentally compatible manner). The pull-on garment is also preferably "absorbent" to absorb and contain the various exudates discharged from the body. A preferred embodiment of the absorbent article is the disposable absorbent pull-on garment, shown in Figure 1.

As used herein, "seam margin" refers to the distance between a distal point of a graphic on a first graphic absorbent article component and an overlapping edge of a second graphic absorbent article component.

The term "substrate" is used herein to describe a material that is primarily two-dimensional (i.e., in an XY plane) and whose thickness (in a Z direction) is relatively small (i.e. 1/10 or less) in comparison to its length (in an X direction) and width (in a Y direction). Non-limiting examples of substrates include a web, layer or layers of fibrous materials, nonwovens, and films and foils, such as polymeric films or metallic foils, for example. These materials may be used alone or may comprise two or more layers laminated together. As such, a web may be a substrate or may be a laminate of two or more substrates.

As used herein, "viewable inner surface" refers to the viewable body-facing surface (e.g., topsheet. The viewable inner surface may comprise multiple layers).

As used herein, "viewable outer surface" refers to the outer facing surface of an absorbent article which is viewable when the absorbent article is worn (e.g., the garment facing side of the outer cover). The viewable outer surface may comprise multiple layers.

As used herein, "whole graphics" refers to a complete graphic, uninterrupted by absorbent article component finishing or one absorbent article component overlapping another.

### ARTICLE

Many existing absorbent pants are structured such that a backsheet and topsheet of a central chassis structure extend to, and from, the front and rear waist edges of the pant in the regions near the wearer's navel in the front, and small of the back in the rear. Separate and discrete side/hip panels are joined to longitudinal (side) edges of the central chassis structure in its front and rear regions, joining them to form the pant structure.

An alternate configuration for absorbent pants is one in which the central chassis structure does not extend to, or form, the front and rear waist edges of the pant. Rather, an elasticized belt structure entirely encircles the wearer's waist and forms the waist edge about the entire pant, and the side/hip panels. The central chassis is joined to the belt structure, usually on the inside thereof, with its ends disposed at locations in the front and rear waist regions somewhat below the waist edges of the belt structure. The elastic belt is usually relatively wide (in the longitudinal direction) and elastically stretchable in the lateral direction. It entirely encircles the wearer's waist, and thereby covers a relatively large amount of the wearer's skin. This configuration is sometimes known as a "belt" or "balloon" configuration (hereinafter, "belt" configuration).

Fig. 1 is a general simplified perspective depiction of a disposable absorbent pant 10 having a belt configuration. Pant 10 may include a central chassis 20 and a belt structure 30. Belt structure 30 may be elastically extensible in the lateral direction, providing elastic stretchability for ease of donning, and a snug and comfortable fit following donning. Central chassis 20 may include a wearer-facing, liquid permeable topsheet (not specifically shown in Fig. 1), an outer- or garment-facing backsheet (not specifically shown in Fig. 1) and an absorbent core (not specifically shown in Fig. 1) sandwiched or enveloped between the topsheet and backsheet. A pair of laterally opposing, longitudinally extending barrier cuffs 25 also may be included with the central chassis in a crotch region thereof, disposed adjacent to the topsheet. Generally the central chassis and barrier cuffs may have any construction and components, including leg cuff structures, suitable for disposable diapers, training pants, and adult incontinence pants, such as, but not limited to, those described in U.S. Patent No. 8,939,957 and application(s) claiming priority thereto.

Belt structure 30 may have a front portion 31 and a rear portion 32. Front and rear portions 31, 32 may be joined together at respective left and right side seams 33L, 33r. Belt structure 30 may form front and rear waist edges 11, 12 defining waist opening 15, and at least portions of left and right leg opening edges 131, 13r of the pant 10.

In Fig. 1, the belt 30 stops short of the crotch region 45 of the pant, at lower edge seam 29. The crotch region 45 is defined as the part of the central chassis 20 that does not overlap either the front or rear portions.

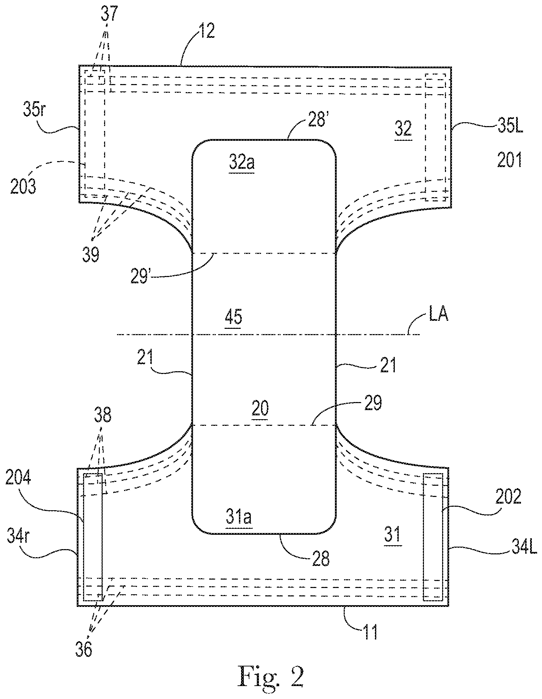

Fig. 2 is a simplified plan view of the precursor structure of the pant 10 shown in Fig. 1, shown prior to joining of front and rear portions 31, 32 along their respective side edges 34L, 35L and 34r, 35r. The pant inside surface is shown. The front lower edge seam 29 and rear lower edge seam 29' form the boundaries of the crotch region 45. The lower edge seams 29 and 29' are formed where the front and rear belt portions overlap with the central chassis closest to the lateral axis LA. The lower edge seams 29 and 29' are on the outside surface of the article. Front region 31a, including front portion 31, and rear region 32a, including rear portion 32, may each include anywhere from 25 percent to 40 percent of the overall longitudinal length of the precursor structure; correspondingly, the crotch region 45 may include anywhere from 20 percent to 50 percent of the overall longitudinal length of the precursor structure, with at least a portion thereof lying at lateral axis LA. Front upper edge seam 28 and rear upper edge seam 28' are formed on the inside surface of the article, where the front and rear belt portions overlap with the central chassis farthest away from the lateral axis LA.

To form pant 10, the precursor structure may be folded along lateral axis LA to bring front and rear regions 31a, 32a, and front and rear portions 31, 32 together such that their side edges 34L, 35L and 34r, 35r, respectively, may be joined at seams 33L, 33r (as shown in Fig. 1). Seams 33L, 33R may be formed by adhesive, thermal, pressure, or ultrasonic bonding, and combinations thereof. In an alternative example, the seams may be formed by mechanical fasteners such as cooperating pairs of hook-and-loop fastening components disposed along side edges 34r, 35r and 34L, 35L, such that the seams are refastenable. Fasteners may also include tape tabs, interlocking fasteners such as tabs & slots, buckles, buttons, snaps, and/or hermaphroditic fastening components.

The article, in some cases the chassis, may have a liquid permeable topsheet forming at least a portion of its inner, wearer-facing surface. The topsheet may be formed of a nonwoven web material which is preferably soft and compatible with sensitive skin, and may be formed of and have any of the features of topsheets used in disposable diapers, training pants and inserts including those described in, for example, US2012/0022485A1. The chassis 20 may also have an outward-facing backsheet, which may be liquid impermeable. The backsheet may be formed of and have any of the features of backsheets used in disposable diapers and training pants including those described in, for example, the U.S. patent application referenced immediately above. Chassis 20 may also have an absorbent core disposed between the topsheet and backsheet. The absorbent core may include one or more absorbent acquisition, distribution and storage material layers and/or components; it may be formed of and have any of the features of absorbent cores used in disposable diapers and training pants including those described in, for example, the U.S. patent application referenced immediately above. The belt structure may be formed of layers of nonwoven web which respectively form inner and outer layers of the belt and the layers of nonwoven web may sandwich one or more elastic members such as a plurality of strands of an elastomeric material. Suitable nonwoven web materials and suitable elastic materials that may be useful in the present invention include those described in US2016/0317695A1. As suggested in Fig 2, the chassis 20 may be affixed to a belt structure 30, to the inner, wearer-facing side thereof. Chassis 20 may be bonded to the belt structure 30 by adhesive, by thermal bonds/welds, mechanical fasteners or a combination thereof. The belt structure may be referred to as flaps Some of these article components are discussed in more detail below.

### GRAPHIC OBJECTS

Absorbent article components may comprise one or more graphics, and may more particularly be referred to as "graphic absorbent article components". Graphics may include, but are not limited to, letters, numbers, symbols, icons, mammal representations, animal representations, insect representations, fish representations, vehicle representations, geometric shapes (e.g., circles, triangles, squares, rectangles, straight and wavy lines, etc.), animations, photographic images, plant representations, landscape representations, patterns (symmetrical or random), textile-like prints or patterns, foliage representations, anthropomorphic representations, as well as those graphics described in U.S. Pat. Pub. No. 2006/0247594. Additionally, graphics may be instructional.

Graphics may be applied to absorbent article components using a number of printing techniques and processes, including, but not limited to, relief printing (including letterpress and flexography), planographic printing (including offset lithography, screenless lithography, collotype, and waterless printing), intaglio printing (including gravure, steel-die, and copper-plate engraving), stencil and screen printing, and electronic printing (including electrostatic, magnetographic, ion or electron deposition, and ink-jet printing). Graphics may be applied to absorbent article components in the absorbent article component's relaxed or stretched state (in the case of stretchable, elastic, or extensible graphic absorbent article components, as further described in U.S. Pat. No. 5,612,118.

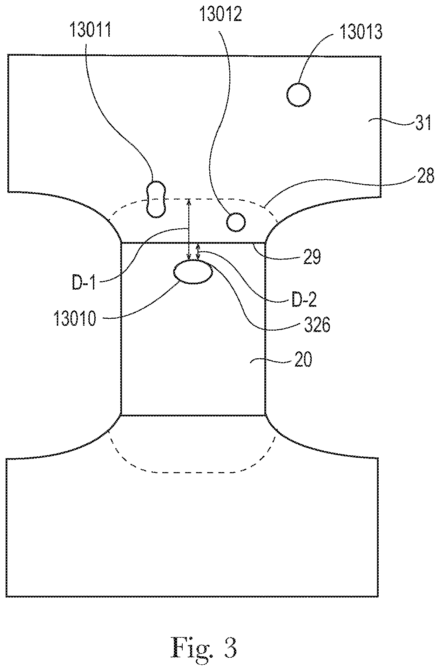

Figure 3 is a schematic showing the front belt portion 31 as it overlaps the central chassis 20. The view is looking at the outside surface of the article and shows the lower edge seam 29 and the upper edge seam 28. A first graphic object 13010 is on the central chassis, with a distal edge 326.

The impact of the seams, for example the lower edge seam and/or the upper edge seam, may be lessened or de-emphasized by minimizing the distance between a particular graphic and a seam. In Figure 3, D-1 represents the distance between the distal edge of the first graphic object and the upper edge seam. D-1 may be from about 50 mm to about 150 mm. D-2 represents the distance between the distal edge of the first graphic object and the lower edge seam. D-2 may be from about 2 mm to about 10 mm, in some embodiments D-2 may be about 5 mm.

The first graphic object is a wetness indicator, such as those described in US filings 14/037,404, 62/147,258, 14/819,501, and 14/663,480. Wetness indicators may be printed on the inner surface (absorbent core side) of the impermeable layer (backsheet film).

Figure 3 also shows second graphic objects 13011, 13012, and 13013 placed entirely on the front belt portion. Some graphic objects may be placed so that no part of the graphic object overlaps or touches a seam, such as 13012 or 13013. Graphic object 13013 is placed such that it does not overlap the central chassis at all. Graphic object 13012, though placed entirely on the front belt portion, does overlap the central chassis, the central chassis being behind the front belt portion. Graphic object 13011, while also being entirely placed on the front belt portion, only partially overlaps the central chassis.

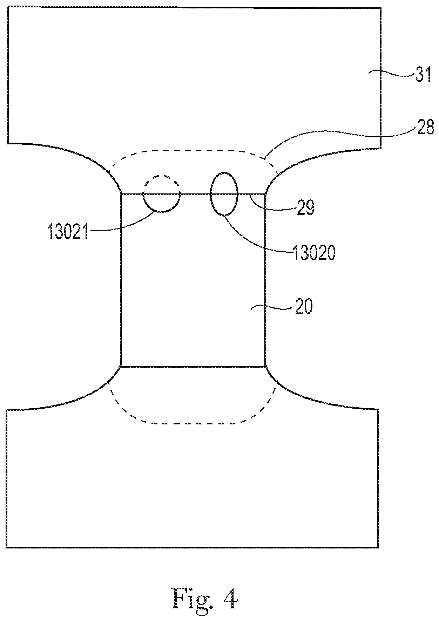

Figure 4 shows a similar outside surface view of an article with graphic objects that overlap the lower edge seam 29. Graphic object 13020 is entirely viewable, ie., is entirely placed on the outside surface of the article, even though part is placed or printed on the central chassis and part on the front belt portion. Graphic object 13021, however, is placed or printed entirely on the central chassis. Because the front belt portion is then placed on top of the central chassis, graphic object 13021 is partially obscured from view.

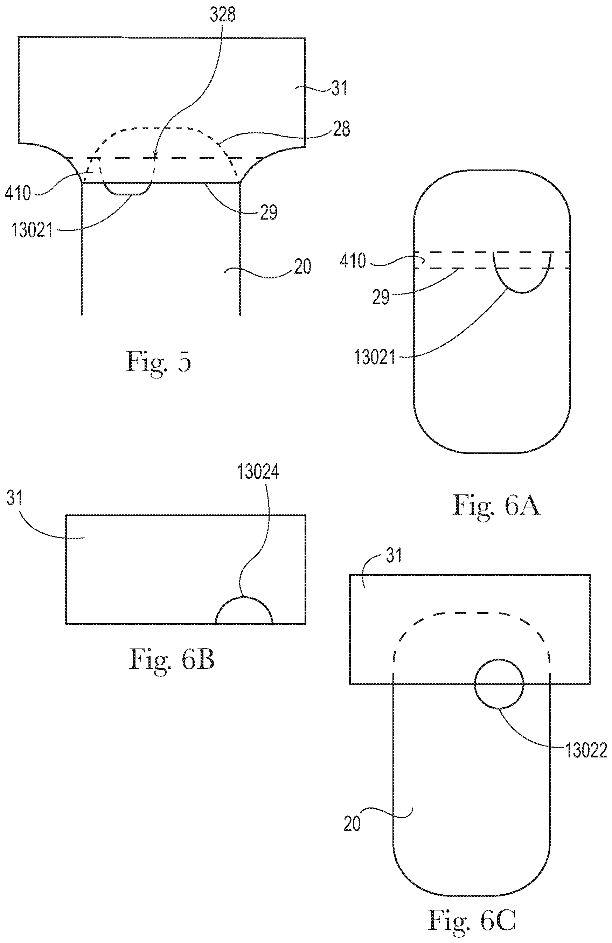

Figure 5 depicts the top part of an article such as shown in figure 3, that is, the front belt portion 31 as it overlaps the central chassis 20, looking at the outside surface of the article. As can be seen in figure 5, in some cases, a graphic object, such as graphic object 13022, may be printed on the central chassis so that a distal point 328 of what is printed on the central chassis is from about 2 mm to about 10 mm above the lower edge seam, ie., farther away from the lateral axis than the lower edge seam. In figure 5, buffer zone 410 depicts this distance. Printing on the central chassis from about 2 mm to about 10 mm above the lower edge seam assures that there will be no gap in the graphic due to the normal variance in the printing process. As shown in Figure 6a, which depicts a central chassis, such as from figures 3 or 4, part of a graphic object 13023 may be printed on the central chassis, about 2 mm to about 10 mm of it in the buffer zone 410 that will be covered over by placement of the front belt portion. Figure 6b shows a graphic object 13024 printed on the front belt portion, wherein graphic object 13024 corresponds and lines up with graphic object 13023 of figure 6a when the front belt portion of figure 6b is placed on top of the central chassis of figure 6a to form the complete graphic object 13022 in figure 6c.

In some embodiments, the upper edge seam 28 may be defined only by the chassis film layer, as the outer nonwoven of the chassis at the upper edge seam may be shorter than the chassis film layer. The upper edge seam 28 is ideally as close as possible to the top of the buffer zone. But in any case the chassis outer nonwoven would not be lower than the upper edge seam, so as to not compromise the appearance of the graphic object. Similarly, in some embodiments, the inner nonwoven belt may be shorter than the outer nonwoven belt, that is, may not extend as far from the lateral axis as the outer nonwoven belt.

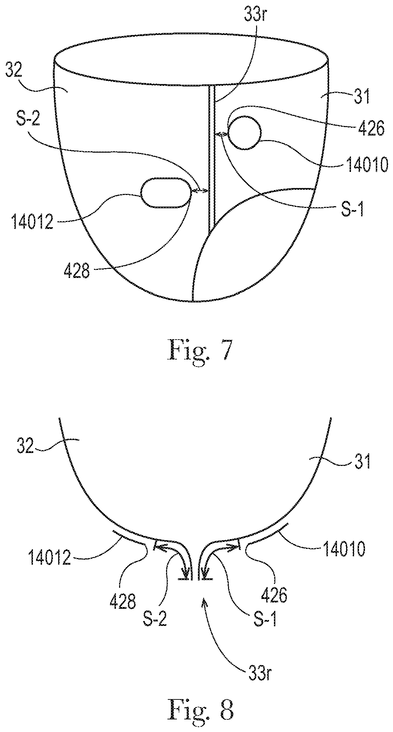

Figure 7 is a schematic of a balloon pant from the side. The side seam 33r is where the front belt portion 31 and the back belt portion 32 are bonded together. A graphic object 14010 with a distal edge 426 is on the front belt portion and a graphic object 14012 with a distal edge 428 is on the rear belt portion. The distance S-1 is the distance between the distal edge 426 of graphic object 14010 and the side seam 33r. The distance S-2 is the distance between the distal edge 428 of the graphic object 14012 and the side seam 33r. By minimizing S-1 and/or S-2, the appearance and presence of the side seam can be less obvious. S-1 and S-2 may be from about 0 mm to about 10 mm, in some embodiments, from about 2 mm to about 10 mm.

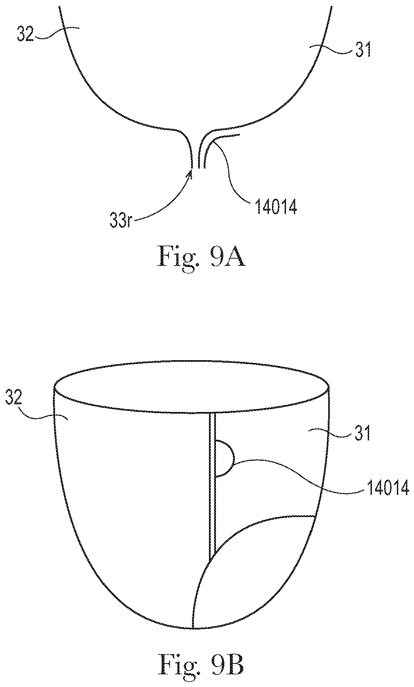

Figure 8 depicts the pant shown in figure 7 from a different perspective. Side seam 33r is formed by bringing the front belt portion 31 and the back belt portion 32 together. Rather than an overlapping seam, like the lower edge seam for example, this side seam is formed by the front and rear belt portions abutting each other. As shown in figure 8 looking down at the top of the article, the front belt portion 31 and rear belt portion 32 are brought together and overlap only slightly in a direction outward from the article, forming side seam 33r. The graphic object 14012 is printed on the rear belt portion. The distance S-2 from a distal edge 428 of the graphic object 14012 to the far side of the side seam 33r may be from about 0 mm to about 10 mm, in some embodiments from about 2 mm to about 10 mm. Similarly, the distance S-1 from the distal edge 426 of the graphic object 14010 to the far side of the side seam 33r may be from about 0 mm to about 10 mm, in some embodiments, from about 2 mm to about 10 mm. Both S-1 and S-2 can be divided into two portions (not shown in figures), wherein the first portion is on the belt portion (front or rear respectively) and the second portion is on the abutted portion of the side seam. The second portions may not be easily visible, as they may not be flush or flat on the body of the wearer of the article. In some embodiments, the first portion of graphic object 14012 may be designed to appear continuous with the first portion of graphic object 14010. The length of the first portions of S-1 and S-2 may be from about 0 mm to about 10 mm. Figure 9a shows a similar perspective view of a pant as figure 8. In figure 9a, the graphic object 14014 is printed on the front belt portion 31 and also on the abutted portion of the side seam 33r. Figure 9b shows the same pant as figure 9a from the side perspective. Graphic object 14014 is only visible on the front belt portion from this point of view.

It may be desirable for embodiments to have graphic objects such as those disclosed in U.S. application 11/999,229. Specifically, the patterns and arrangements of the graphic objects disclosed in U.S. 11/999,229 may be incorporated into the present invention. The noticeability rating as described in U.S. 11/999,229 may also be used to measure the seam noticeability with the particular graphic objects.

A graphic object may be printed on the inner surface of the outer belt, that is, on the inner surface of the outer nonwoven of the belt or on the inner surface of the outer nonwoven of the chassis. A graphic object may also or alternatively be placed on the outer surface of the impermeable layer (backsheet film) of the chassis. The graphic object may be printed after the backsheet nonwoven and the film are laminated. The first graphic object is a wetness indicator, which may be printed on the inner surface of the backsheet film, wherein the wetness indicator is in liquid communication with the absorbent core.

Suitable methods of printing graphics and assembling article components with graphics may be found in U.S. applications 12/476,348, 14/635,189, 62/093,452, 62/093,516, 62/093,604, 62/093,620, 62/093,438, 62/147,004, and 62/147,006.

### TOPSHEET

In one embodiment, the absorbent article may comprise a topsheet. The topsheet may be compliant, soft feeling, and non-irritating to the wearer's skin and may be elastically stretchable in one or more directions. Further, the topsheet may be liquid pervious, permitting liquids (e.g., menses, urine, and/or runny feces) to penetrate through its thickness. Various topsheets may also comprise a hydrophilic material, for example, which is configured to draw bodily fluids into an absorbent core of the chassis when these fluids are expelled from the body. A suitable topsheet may be manufactured from a wide range of materials, such as woven and nonwoven materials, apertured or hydroformed thermoplastic films, apertured nonwovens, porous foams, reticulated foams, reticulated thermoplastic films, and/or thermoplastic scrims, for example. Suitable apertured films may comprise those described in U.S. Pat. Nos. 3,929,135, 4,324,246, 4,342,314, 4,463,045, 5,006,394, 5,628,097, 5,916,661, 6,545,197, and 6,107,539.

Apertured film or nonwoven topsheets typically may be pervious to bodily exudates, yet non-absorbent, and have a reduced tendency to allow fluids to pass back through and rewet the wearer's skin. Suitable woven and nonwoven materials may comprise natural fibers, such as, for example, wood or cotton fibers, synthetic fibers, such as, for example, polyester, polypropylene, or polyethylene fibers, or combinations thereof. If the topsheet 81 comprises fibers, the fibers may be spunbond, carded, wet-laid, meltblown, hydroentangled, or otherwise processed, for example, as is generally known in the art.

The topsheet may comprise a skin care lotion. Examples of suitable lotions include, but are not limited to, those described in U.S. Pat. Nos. 5,607,760; 5,609,587; 5,635,191; 5,643,588; and 5,968,025, and as described in U.S. Application No. 61/391,353, and as described in U.S. Pub. No. 2014-0257216. Beyond these compositions, the absorbent article may comprise soluble cyclodextrin derivatives such as those described in U.S. Pub. No. 2014/0274870.

Additionally, the topsheet of the present disclosure may be a tufted laminate web as disclosed in U.S. Pat. No. 7,410,683, and/or may be an apertured web as disclosed in PCT/CN2014/083769 having an international filing date of August 6, 2014.

In one embodiment, the topsheet may comprise graphics such that depth perception is created as described in U.S. Pat. No. 7,163,528.

### BACKSHEET

In one embodiment, the absorbent article may comprise a backsheet. The backsheet may be impervious, or at least partially impervious, to fluids or body exudates (e.g., menses, urine, and/or runny feces) and may be manufactured from a thin plastic film, although other flexible liquid impervious materials may also be used. The backsheet may prevent the body exudates or fluids absorbed and contained in an absorbent core of the absorbent article from wetting articles which contact the absorbent article, such as bedsheets, pajamas, clothes, and/or undergarments. The backsheet may comprise a woven or nonwoven material, polymeric films such as thermoplastic films of polyethylene or polypropylene, and/or a multi-layer or composite materials comprising a film and a nonwoven material (e.g., having an inner film layer and an outer nonwoven layer). A suitable backsheet may comprise a polyethylene film having a thickness of from about 0.012 mm (0.5 mils) to about 0.051 mm (2.0 mils). Examples of polyethylene films are manufactured by Clopay Corporation of Cincinnati, Ohio, under the designation BR-120 and BR-121, and by Tredegar Film Products of Terre Haute, Ind., under the designation XP-39385.

One suitable material for the backsheet can be a liquid impervious thermoplastic film having a thickness of from about 0.012 mm (0.50 mil) to about 0.051 mm (2.0 mils), for example including polyethylene or polypropylene. Typically, the backsheet can have a basis weight of from about 5 g/m2 to about 35 g/m2. The backsheet can be typically positioned adjacent the outer-facing surface of the absorbent core and can be joined thereto. For example, the backsheet may be secured to the absorbent core by a uniform continuous layer of adhesive, a patterned layer of adhesive, or an array of separate lines, spirals, or spots of adhesive. Illustrative, but non-limiting adhesives, include adhesives manufactured by H. B. Fuller Company of St. Paul, Minn., U.S.A., and marketed as HL-1358J. An example of a suitable attachment device including an open pattern network of filaments of adhesive is disclosed in U.S. Pat. No. 4,573,986. Another suitable attachment device including several lines of adhesive filaments swirled into a spiral pattern is illustrated by the apparatus and methods shown in U.S. Pat. Nos. 3,911,173; 4,785,996; and 4,842,666. Alternatively, the attachment device may include heat bonds, pressure bonds, ultrasonic bonds, dynamic mechanical bonds, or any other suitable attachment device or combinations of these attachment devices.

In one embodiment, the backsheet may be embossed and/or matte-finished to provide a more cloth-like appearance. Further, the backsheet may permit vapors to escape from the absorbent core of the absorbent article (i.e., the backsheet is breathable) while still preventing, or at least inhibiting, fluids or body exudates from passing through the backsheet. In one embodiment, the size of the backsheet may be dictated by the size of the absorbent article and the design or configuration of the absorbent article to be formed, for example.

### ABSORBENT CORE

In various embodiments, the absorbent article may comprise an absorbent core (also referred to as an "absorbent member" or "absorbent assembly" or "absorbent structure" or "absorbent composite") that is disposed between the topsheet and the backsheet. The absorbent core may comprise a laterally extending front edge in the front waist region, a longitudinally opposing and laterally extending back edge in the back waist region, a first longitudinally extending side edge, and a laterally opposing and second longitudinally extending side edge. Both of the side edges may extend longitudinally between the front edge and the back edge. In one embodiment, more than one absorbent core or more than one absorbent core layer may be provided in an absorbent article, for example. The absorbent core may be any suitable size or shape that is compatible with the absorbent article. Example absorbent structures for use as the absorbent core of the present disclosure that have achieved acceptance and commercial success are described in U.S. Pat. Nos. 4,610,678; 4,673,402; 4,888,231; and 4,834,735.

In one embodiment, suitable absorbent cores may comprise cellulosic airfelt material. For instance, such absorbent cores may comprise less than about 40%, 30%, 20%, 10%, 5%, or even 1% of the cellulosic airfelt material as determined by weight. Additionally, such an absorbent core may be primarily comprised of an absorbent gelling material in amounts of at least about 60%, 70%, 80%, 85%, 90%, 95%, or even about 100% as determined by weight. Furthermore, a portion of the absorbent core may comprise a microfiber glue (if applicable). Such absorbent cores, microfiber glues, and absorbent gelling materials are described in U.S. Pat. Nos. 5,599,335; 5,562,646; 5,669,894; 6,790,798; and 7,521,587 and in U.S. Pat. Publ. No. 2004/0158212.

In one embodiment, the core, including multiple layers making up the core system, may be printed and embossed as described in U.S. Pat. No. 8,536,401.

In one embodiment, the core may be separable from the chassis as disclosed in U.S. Pat. Nos. 6,989,006; 7,381,202; 7,175,613; 7,824,386; 7,766,887; and 6,989,005. In such embodiments, the measurements described in this disclosure may be made to the chassis alone or may be made to the chassis in combination with the separable core/absorbent assembly.

In one embodiment, the absorbent article of the present disclosure, and particularly, a portion where the absorbent member is disposed, may have a body fluid absorption rate greater than 3 g/sec according to US Pat. No. 6,649,810. According to U.S. Pat. No. 6,649,810, the expression "the portion (of the absorbent article) where the absorbent member is disposed" is intended to mean the portion occupied by the absorbent member when the absorbent article is flatly unfolded and seen in its plan view.

In one embodiment, the absorbent structure may have an intake factor greater than 3 according to US Pat. No. 7,073,373, wherein the intake factor is defined as the absorbent core permeability divided by the normalized retention capacity (which is defined by the Retention Capacity Test - also according to U.S. Pat. No. 7,073, 373).

In one embodiment, the absorbent composite has a body fluid absorption greater than 75 g/100 cm2, according to U.S. Pat. No. 6,649,810.

In one embodiment, a target location of the absorbent article may have a wicking value greater than 36%, according to U.S. Pat. No. 6,383,960.

In one embodiment, the absorbent article may have a bending stiffness between 0.05-1.0 gf, according to U.S. Pat. No. 5,810,796.

In one embodiment, the absorbent article may have a crotch fluid absorption rate greater than 3g/sec according to U.S. Pat. No. 6,649,810. In one embodiment, a freeze-dried composite of the absorbent composite may have an intake rate of at least about 1.9 cubic centimeters (cc) of liquid/second at 80% composite saturation according to U.S. Pat. No. 6,689,934.

In some embodiments the absorbent core may comprise channels as described in U.S. Pat. No. 8,568,566; U.S. Pub. Nos. 2012/316046, 2014/027066, 2014/163500, 2014/163506, 2014/163511, 2012/316526, 2012/316527, 2012/316528, 2012/316529, 2012/316523, 2014/163501, 2014/163502, 2014/163503 and European Pub. Nos. 2532328, 2532329, 2717823, 2717820, 2717821, 2717822, 2532332, 2740449, and 2740452.

In some embodiments the absorbent layer may comprise at least two channels substantially free of absorbent polymer particles extending through the thickness of the absorbent layer in the longitudinal dimension of the absorbent layer. By extending in the longitudinal dimension of the absorbent layer, it is meant that the channels extend essentially in the longitudinal dimension, i.e. they extend more in the longitudinal dimension than in the transverse dimension, e.g. at least twice as much in the longitudinal dimension than in the transverse dimension.

"Channels" as used herein refer to discrete portions of one or more of the absorbent layers of the absorbent core extending through the thickness of the absorbent layer which are substantially free of absorbent polymer (particles or fibers), i.e., no absorbent polymer particles are intentionally present in such a channel (longitudinal main channel or secondary channel) of an absorbent structure. However, it should be understood that, accidentally, a small, negligible amount of absorbent polymer particles may be present in the channel, which may not contribute to any significant degree to the overall functionality (e.g. absorbency of the absorbent structure). Typically, the channels possess two transverse edges (in the shortest dimension) and two longitudinal edges (in the longest dimension) running between the transverse edges. The transverse edges of the channels may be straight (i.e,. perpendicular to the longitudinal side edges), angled or curved. The channels may have an average width w of at least 3 mm (the average of a channel is defined as the average distance between the longitudinal side edges) or may have at least 4% of the width of the absorbent layer.

The channels may be permanent. By permanent, it is meant that the integrity of the channels is at least partially maintained both in dry state and wet state, i.e., the channels are resistant to external forces caused by movements of the diaper's wearer. Permanent channels are obtained by immobilizing the absorbent polymer on the substrate layer, such as by applying a thermoplastic adhesive material over the absorbent layer. The absorbent layer of the present disclosure may comprise in particular permanent channels formed by bonding of a first substrate layer and a second substrate layer through the channels. Typically, glue may be used to bond both substrate layers through the channel, but it is possible to bond via other known means, for example ultrasonic bonding, pressure bonding or thermal bonding. The supporting layers can be continuously bonded or intermittently bonded within the channels.

In some embodiments, it may be desirable to have an array of articles comprising absorbent cores with channels, such as those disclosed in 62/104,330.

### LEG CUFFS

In one embodiment, the chassis of the absorbent article may comprise longitudinally extending and laterally opposing leg cuffs and that are disposed on the interior surface of the chassis that faces inwardly toward the wearer and contacts the wearer. The leg cuffs and may comprise one or more elastic gathering members disposed at or adjacent the proximal edge of one or both of the leg cuffs. In addition, the elastic gathering members of the leg cuff may also comprise one or more elastic strands disposed at or adjacent the distal edge of one or both of the leg cuffs. The elasticized leg cuffs may comprise several embodiments for reducing the leakage of body exudates or fluids in the leg regions. The elasticized leg cuffs are sometimes referred to as leg bands, barrier cuffs, elastic cuffs, or gasketing cuffs. Suitable elasticized leg cuffs may comprise those described in U.S. Pat. Nos. 3,860,003, 4,909,803, 4,695,278, 4,795,454, 4,704,115, and 4,909,803, and U.S. Pat. Publ. No. 2009/0312730. The leg cuffs may be formed by folding portions of the chassis laterally inward, i.e., toward the longitudinal axis, to form both the respective leg cuffs and the side edges of the chassis. In other embodiments, the leg cuffs may be formed by attaching an additional layer or layers to the chassis at or adjacent to each of the respective side edges of the chassis. In one embodiment, the chassis may also comprise other elastics disposed adjacent the side edges which may cause the article to form into a "U" shape when allowed to relax thereby pulling the interior surface of the front waist region toward the interior surface of the back waist region.

In one embodiment, each leg cuff may comprise a proximal edge. These edges are positioned proximate to the longitudinal axis compared to distal edges. The leg cuffs may overlap the absorbent core, i.e., the proximal edges lie laterally inward of the respective side edges and of the absorbent core. Such an overlapped configuration may be desirable in order to impart a more finished appearance to the absorbent article than that imparted by a non-overlapped configuration. In other embodiments, the leg cuffs may not overlap the absorbent core.

In one embodiment, each leg cuff may be attached to the interior surface of the chassis in a leg cuff attachment zone (not shown) adjacent to the front waist end edge and in a longitudinally opposing leg cuff attachment zone (not shown) adjacent to the back waist end edge. In one embodiment, between the leg cuff attachment zones, the proximal edge of the leg cuff remains free, i.e., not attached to the interior surface of the chassis or to the absorbent core. Also, between the longitudinally opposing leg cuff attachment zones, each leg cuff may comprise one or more (specifically including one, two, three, or four elastic strands per leg cuff) longitudinally extensible cuff elastic gathering members that may be disposed at or adjacent to the proximal edge of the leg cuff by any suitable methods. Each of such cuff elastic gathering members may be attached over the leg cuffs entire length or over only a portion of the leg cuffs length. For example, such cuff elastic gathering members may be attached only at or near the leg cuffs longitudinally opposing ends and may be unattached at the middle of the leg cuffs length. Such cuff elastic gathering members may be disposed in the crotch region and may extend into one or both of the front waist region and the back waist region. For example, an elastic gathering member may be attached at or adjacent to the proximal edge of each of the leg cuffs and extends into both the front waist region and the back waist region.

In various embodiments, each cuff elastic gathering member may be enclosed inside a folded hem for example. In various embodiments, the cuff elastic gathering members may be sandwiched between two layers forming the leg cuff, by two layers of the chassis, or may be attached on a surface of the chassis or the leg cuff and remain exposed.

In one embodiment, when stretched, the cuff elastic gathering member disposed adjacent to each leg cuffs proximal edge allows the leg cuff proximal edge to extend to the flat uncontracted length of the chassis, e.g., the length of the chassis. When allowed to relax, the cuff elastic gathering member contracts to pull the front waist region and the back waist region toward each other and, thereby, bend the article into a "U" shape in which the interior of the "U" shape may be formed by the portions of the article that are intended to be placed toward the body of the wearer (i.e., interior surface). Because each of the proximal edges remains free between the longitudinally oriented leg cuff attachment zones, the contractive force of the elastic gathering member may lift the proximal edge of the leg cuff away from the interior surface of the chassis. This lifting of the proximal edges when the article is in the relaxed condition lifts the leg cuffs into a position to serve as side barriers to prevent, or at least inhibit, leakage of bodily exudates.

Examples of acceptable leg cuffs are disclosed in U.S.S.N. 13/457,521, filed April 27, 2012, including the configurations disclosed by Figures 8a-t of the ' 521 application.

### WAISTBAND

In one embodiment, the article may comprise an elasticized waistband. The elasticized waistband may provide improved fit and containment and may be configured to elastically expand and contract laterally to dynamically fit a wearer's waist. The elasticized waistband may extend longitudinally from the waist edge of the absorbent article toward the waist edge of the absorbent core. In one embodiment, the absorbent article may have two elasticized waistbands, one positioned in the back waist region and one positioned in the front waist region, although other pant embodiments may be constructed with a single elasticized waistband. The elasticized waistband may be constructed in a number of different configurations including those described in U.S. Pat. Nos. 4,515,595 and 5,151,092.

In one embodiment, the elasticized waistbands may comprise materials that have been "prestrained" or "mechanically prestrained" (i.e., subjected to some degree of localized pattern mechanical stretching to permanently elongate the material). The materials may be prestrained using suitable deep embossing techniques. In other embodiments, the materials may be prestrained by directing the material through an incremental mechanical stretching system as described in U.S. Pat. No. 5,330,458. The materials may then be allowed to return to their substantially untensioned condition, thus forming a zero strain stretch material that is extensible, at least up to the point of initial stretching. Examples of zero strain materials are disclosed in U.S. Pat. Nos. 2,075,189, 3,025,199, 4,107,364, 4,209,563, 4,834,741, and 5,151,092.

### FLAPS

The flaps may be discrete from or integral with the chassis. A discrete flap is formed as separate element, which is joined to the chassis. In some embodiments this may include a front and/or back belt-like flaps ("belts") being joined across the front and back (or rear) waist regions of the chassis, at least across end edges of the chassis. In some embodiments the waistbands can overlap the flaps to create a continuous belt-like structure.

The belt-like flaps may comprise an inner nonwoven layer and an outer nonwoven layer and elastics there between. The inner and outer nonwoven layers may be joined using adhesive or thermoplastic bonds. Various suitable belt-like flap configurations can be found in US2013/0211363.

An integral flap is a portion, one or more layers, of the chassis that projects laterally outward from the longitudinal edge. The integral flap may be formed by cutting the chassis to include the shape of the flap projection.

While many of the embodiments illustrated in this application having belt-like flaps are pant articles, taped articles may have belt-like flaps disposed in one or both waist regions as well.

The structure of flaps play an important role in the functionality of the absorbent article and are fundamentally different than the elastics used in underwear. As mentioned above, incontinence events, such as SUI and UUI, can result in a high flow rate and/or a full bladder release. The amounts of urine expelled during the incontinence events can vary wildly given the type of urinary incontinence as well as other circumstances such as time since last bathroom visit, amount of fluid intake, day or night, etc. Loadings can range from as low as a few drops of urine to loadings as high as 600 mls. It is not unusual to have single loadings as high as 300, 400 and even 500 mls. These levels of loading present a significant downward force associated with the loading which can be a pound or more. This downward force must be compensated for by the absorbent article chassis in order to minimize sagging, gapping and leakage. In order to sustain the fit of the article even after loading the article comprises elastomeric element(s), including films and/or strands that are disposed proximate to and along the side seams of the article and extend laterally from one side toward the other. These elastomeric element(s) should create a normal force against the body sufficient to anchor the article. The location of the elastomeric element(s), as well as the forces exerted by the elastomeric element(s) can be varied to ensure proper anchoring at the hips and along the body specifically across the front waist region and in the back waist region. One form of anchoring beneficial for sustaining the fit of a loaded article is disclosed in U.S. Pat. No. 5,358,500 Absorbent Articles Providing Sustained Dynamic Fit issued Oct 25, 1994 to LaVon, et al. It should also be noted that regular underwear with elastic along the waist edge and leg edges would not typically provide sufficient support to sustain the fit of the underwear if a weight of 300-600 grams was applied to the crotch region of the underwear. This paragraph illustrates another fundamental reason why proper Body Rise/Length of the absorbent article is key to maintain contact and gasketing, as well as proper anchoring on the body to overcome the fit degrading forces associated with high loadings.

The seams may each be from about 150 mm to about 200 mm, from about 160 mm to about 190 mm, or from about 170 mm to about 180 mm. The seams are the portions of the flap that overlap (i.e., the distance from the waist opening to the leg opening of the overlapped or abutted flaps).

### FASTENING SYSTEM

The absorbent article may also include a fastening system. When fastened, the fastening system interconnects the front waist region and the rear waist region resulting in a waist circumference that may encircle the wearer during wear of the absorbent article. The fastened elements connecting the front and back waist regions form refastenable side seams. This may be accomplished by flaps in the back waist region interconnecting with flaps in the front waist region or by flaps in the back waist region interconnecting with the chassis in the front waist region. The fastening system may comprises a fastener such as tape tabs, hook and loop fastening components, interlocking fasteners such as tabs & slots, buckles, buttons, snaps, and/or hermaphroditic fastening components, although any other known fastening means are generally acceptable. The fasteners may releasably engage with a landing zone, which may be a woven or nonwoven. Some exemplary surface fastening systems are disclosed in U.S. Patent Nos. 3,848,594; 4,662,875; 4,846,815; 4,894,060; 4,946,527; 5,151,092; 5,221,274. Particularly, the flaps may be configured as described and illustrated in Figs. 3A-C and 4A-k of U.S.S.N. 61/666,065, filed on June 29, 2012, titled DISPOSABLE ABSORBENT REFASTENABLE PANTS AND METHODS FOR MANUFACTURING THE SAME Further, the absorbent articles of this disclosure may be manufactured in accordance with the descriptions and illustrations of U.S.S.N. 61/666,065 (see, for example, Figs. 5-10C of the '065 application). An exemplary interlocking fastening system is disclosed in U.S. Patent No. 6,432,098. The fastening system may also provide a means for holding the article in a disposal configuration as disclosed in U.S. Pat. No. 4,963,140. The fastening system may also include primary and secondary fastening systems, as disclosed in U.S. Pat. No. 4,699,622. The fastening system may be constructed to reduce shifting of overlapped portions or to improve fit as disclosed in U.S. Patent Nos. 5,242,436; 5,499,978; 5,507,736; and 5,591,152.

In some embodiments, a refastenable system may be used such as those disclosed in U.S. applications US2014/0005628A1 and US2014/0005020A1 .

### IDENTICAL OR SUBSTANTIALLY IDENTICAL CHASSIS

As disclosed in U.S. Pub. No. 2013-0211355, it may be desirable to offer an array of packages for fitting different sized wearers, but comprising identical or substantially identical chassis. For instance, an array may comprise a first package comprising a first size of absorbent articles and a second package may comprise a second size of absorbent articles, where the first and second packages comprise identical or substantially identical chassis as described in U.S. Pub. No. 2013-0211355. More particularly, the first package may comprise a first chassis and the second package may comprise a second chassis, where each of the first and second chassis comprise the same dimensions of one or more of: core width at the lateral centerline, core width at one of the front or rear core end, a distance from a left outer cuff distal edge to a right outer cuff distal edge, a distance from a left inner cuff distal edge to a left outer cuff distal edge, a distance from a left inner cuff proximal edge to a right inner cuff proximal edge, a distance from a left inner cuff proximal edge to a left outer cuff distal edge, a free height of the inner cuff, inner cuff hem fold width, inner cuff elastics length, outer cuff elastics length, core length, and backsheet width.

Further, each of the first and second chassis may comprise identical chemical compositions of one or more of a topsheet, backsheet film, backsheet nonwoven, core super absorbent polymers, core pulp, core nonwoven, core tissue, leg cuff film, leg cuff nonwoven, super absorbent polymer adhesive, core nonwoven adhesive, leg cuff elastic adhesive, and backsheet nonwoven/film adhesive.

And, each of the first and second chassis may comprise the same basis weight of one or more of the topsheet, backsheet film, backsheet nonwoven, core super absorbent polymers, core pulp, leg cuff nonwoven, leg cuff film, super absorbent polymer adhesive, leg cuff adhesive, and backsheet nonwoven/film adhesive.

And, each of the first and second chassis may comprise compositionally identical core super absorbent polymers. The first and second chassis may have identical component cross sectional order and disposition in at least one of the front waist region, back waist region, and crotch region. The inner leg cuffs of the first and second chassis may be composed of the compositionally identical materials.

And, the core adhesives of the first and second chassis may be the same adhesive(s). The first and second chassis may comprise core super absorbent polymers that are in the same chemical class and subclass.

And, each of the first and second chassis may comprise first and second wetness indicators, respectively, and wherein the first and second wetness indicators are compositionally identical.

Further, the inner leg cuffs of the first and second chassis may have identical component cross sectional order and disposition in at least one of the front waist region, back waist region, and crotch region. The distance from the left outer cuff distal edge to a right outer cuff distal edge may the same. The distance from the left inner cuff proximal edge to left outer cuff distal edge may be the same. The distance from the left inner cuff proximal edge to the right inner cuff proximal edge is the same. The lengths of the inner and outer cuffs are the same.

In some embodiments, different size offerings in an array may have identical or substantially identical chassis as the flaps or belts may be used to enable the absorbent article to fit different sized wearers. For example, first and second absorbent articles may have identical chassis (compositionally, dimensionally, cross-sectionally), but the first article may have a different length due to disposition of the belts, such that the first article may be targeted to fit a smaller wearer than the second article. As a second example, first and second absorbent articles may have identical chassis (compositionally, dimensionally, cross-sectionally), but the first article may have a different length and/or width due to the size of the belts, such that the first article may be targeted to fit a smaller wearer than the second article.

In some embodiments, first and second absorbent articles may have identical chassis compositionally, but not dimensionally, and not cross-sectionally. In some embodiments, first and second absorbent articles may have identical chassis dimensionally, but not compositionally, and not cross-sectionally. In some embodiments, first and second absorbent articles may have identical chassis cross-sectionally, but not dimensionally, and not compositionally. In still other embodiments, first and second absorbent articles may have two, but not three of (1) compositionally, (2) dimensionally, and (3) cross-sectionally identical chassis.

## Claims

1. An absorbent article (10) having an outside surface and an inside surface; a front region, a rear region, and a crotch region disposed therebetween; and comprising:
a liquid permeable topsheet; a backsheet, and an absorbent core disposed between the top sheet and the backsheet;
a central chassis (20) occupying the crotch region and comprising left and right longitudinal edges, and further comprising a first graphic object (13010);
a belt structure (30) disposed about the central chassis, the belt structure overlaying the backsheet to the outside thereof in the front and rear regions, the belt structure overlapping and extending laterally and longitudinally outward from the chassis, forming a lower edge seam (29) on the article outside surface where the belt structure overlaps the central chassis closest to the lateral axis and an upper edge seam (28) on the article inside surface where the belt structure overlaps the central chassis farthest away from the lateral axis (LA);
the belt structure having a front portion (31) having a front waist edge (11), and front left and right side edges;
the belt structure having a rear portion (32) having a rear waist edge (12) and rear left and right side edges;
the respective front and rear left side edges and the respective front and rear right side edges being joined at side seams (33r, 331), the belt structure forming a waist opening (15) and left and right leg openings (13r ,131);
wherein the front belt portion comprises a second graphic object (13011, 13012, 13013);
**characterized in that** the first graphic object (13010) is a wetness indicator, and
wherein the distance (D-1) from a distal edge of the first graphic object (13010) to the upper edge seam (28) is from 50 mm to 150 mm;
and wherein the distance (D-2) from a distal edge of the first graphic object (13010) and the lower edge seam (29) is from 2 mm to 10 mm.

2. The absorbent article of claim 1, wherein the first graphic object is printed on the inner surface of the backsheet film.

3. The absorbent article of claim 1, wherein the second graphic object (13013) does not overlap the central chassis.

4. The absorbent article of claim 1, wherein the entire second graphic object (13012) is on the front belt portion and overlaps the central chassis.

5. The absorbent article of claim 1, wherein the second graphic object (13011) partially overlaps the central chassis.

6. The absorbent article of claim 1, wherein the article comprises a second graphic object that is printed partially on the front belt portion and partially on the central chassis.

7. The absorbent article of claim 1, wherein the article comprises a second graphic object that is printed on the central chassis, wherein a distal point of the second graphic object is from 2 mm to 10 mm farther away from the lateral axis than the lower edge seam.

## Patentansprüche

1. Absorptionsartikel (10), die Folgendes aufweist: eine Außenoberfläche und eine Innenoberfläche; einen vorderen Bereich, einen hinteren Bereich und einen dazwischen angeordneten Schrittbereich; und umfassend:
eine flüssigkeitsdurchlässige Oberschicht; eine Unterschicht und einen Absorptionskern, der zwischen der Oberschicht und der Unterschicht angeordnet ist;
eine mittlere Grundeinheit (20), die den Schrittbereich einnimmt und linke und rechte Längsränder umfasst, und ferner ein erstes Grafikobjekt (13010) umfasst;
eine gegurtete Struktur (30), die um die mittlere Grundeinheit herum angeordnet ist, wobei die gegurtete Struktur die Unterschicht zur Außenseite davon in den vorderen und hinteren Bereichen überlagert, wobei die gegurtete Struktur die Grundeinheit überlappt und sich seitlich und in Längsrichtung davon nach außen erstreckt, die eine untere Randnaht (29) auf der Außenoberfläche des Artikels, wo die gegurtete Struktur die mittlere Grundeinheit überlappt, die der Querachse am nächsten ist, und eine obere Randnaht (28) auf der Innenoberfläche des Artikels bildet, wo die gegurtete Struktur die mittlere Grundeinheit überlappt, die am weitesten von der Querachse (LA) entfernt ist;
wobei die gegurtete Struktur einen vorderen Abschnitt (31) mit einem vorderen Taillenrand (11) und vordere linke und rechte Seitenränder aufweist;
wobei die gegurtete Struktur einen hinteren Abschnitt (32) mit einem hinteren Taillenrand (12) und hintere linke und rechte Seitenränder aufweist;
wobei die jeweiligen vorderen und hinteren linken Seitenränder und die jeweiligen vorderen und hinteren rechten Seitenränder an Seitennähten (33r, 33l) verbunden sind, wobei die gegurtete Struktur eine Taillenöffnung (15) und linke und rechte Beinöffnungen (13r, 13l) bildet;
wobei der vordere Gurtabschnitt ein zweites Grafikobjekt (13011, 13012, 13013) umfasst;
**dadurch gekennzeichnet, dass** das erste Grafikobjekt (13010) ein Nässeindikator ist, und
wobei der Abstand (D-1) von einem distalen Rand des ersten Grafikobjekts (13010) zur oberen Randnaht (28) von 50 mm bis 150 mm beträgt;
und wobei der Abstand (D-2) von einem distalen Rand des ersten Grafikobjekts (13010) und der unteren Randnaht (29) von 2 mm bis 10 mm beträgt.

2. Absorptionsartikel nach Anspruch 1, wobei das erste Grafikobjekt auf die Innenoberfläche der Unterschichtfolie gedruckt ist.

3. Absorptionsartikel nach Anspruch 1, wobei das zweite Grafikobjekt (13013) die mittlere Grundeinheit nicht überlappt.

4. Absorptionsartikel nach Anspruch 1, wobei sich das gesamte zweite Grafikobjekt (13012) auf dem vorderen Gurtabschnitt befindet und die mittlere Grundeinheit überlappt.

5. Absorptionsartikel nach Anspruch 1, wobei das zweite Grafikobjekt (13011) die mittlere Grundeinheit teilweise überlappt.

6. Absorptionsartikel nach Anspruch 1, wobei der Artikel ein zweites Grafikobjekt umfasst, das teilweise auf den vorderen Gurtabschnitt und teilweise auf die mittlere Grundeinheit gedruckt ist.

7. Absorptionsartikel nach Anspruch 1, wobei der Artikel ein zweites Grafikobjekt umfasst, das auf die mittlere Grundeinheit gedruckt ist, wobei ein distaler Punkt des zweiten Grafikobjekts von 2 mm bis 10 mm weiter von der Querachse entfernt ist als die untere Randnaht.

## Revendications

1. Article absorbant (10) ayant une surface extérieure et une surface intérieure ; une région avant, une région arrière, et une région d'entrejambe disposée entre elles ; et comprenant :
une feuille de dessus perméable aux liquides ; une feuille de fond, et une âme absorbante disposée entre la feuille de dessus et la feuille de fond ;
un châssis central (20) occupant la région d'entrejambe et comprenant des bords longitudinaux gauche et droit, et comprenant en outre un premier objet graphique (13010) ;
une structure de ceinture (30) disposée autour du châssis central, la structure de ceinture recouvrant la feuille de fond jusqu'à l'extérieur de celle-ci dans les régions avant et arrière, la structure de ceinture chevauchant et s'étendant latéralement et longitudinalement vers l'extérieur à partir du châssis, formant une couture (29) de bord inférieur sur la surface extérieure de l'article où la structure de ceinture chevauche le châssis central le plus proche de l'axe latéral et une couture de bord supérieur (28) sur la surface intérieure de l'article où la structure de ceinture chevauche le châssis central le plus éloigné de l'axe latéral (LA) ;
la structure de ceinture ayant une partie avant (31) ayant un bord de taille avant (11), et des bords latéraux avant gauche et droit ;
la structure de ceinture ayant une partie arrière (32) ayant un bord de taille arrière (12) et des bords latéraux arrière gauche et droit ;
les bords latéraux gauches avant et arrière respectifs et les bords latéraux droits avant et arrière respectifs étant reliés au niveau de coutures latérales (33r, 33l), la structure de ceinture formant une ouverture de taille (15) et des ouvertures de jambe gauche et droite (13r, 13l) ;
dans lequel la partie de ceinture avant comprend un deuxième objet graphique (13011, 13012, 13013) ;
**caractérisé en ce que** le premier objet graphique (13010) est un indicateur d'humidité, et
dans lequel la distance (D-1) d'un bord distal du premier objet graphique (13010) à la couture de bord supérieur (28) va de 50 millimètres à 150 millimètres ;
et dans lequel la distance (D-2) d'un bord distal du premier objet graphique (13010) et de la couture de bord inférieur (29) va de 2 millimètres à 10 millimètres.

2. Article absorbant selon la revendication 1, dans lequel le premier objet graphique est imprimé sur la surface intérieure du film de feuille de fond.

3. Article absorbant selon la revendication 1, dans lequel le deuxième objet graphique (13013) ne chevauche pas le châssis central.

4. Article absorbant selon la revendication 1, dans lequel la totalité du deuxième objet graphique (13012) se trouve sur la partie de ceinture avant et chevauche le châssis central.

5. Article absorbant selon la revendication 1, dans lequel le deuxième objet graphique (13011) chevauche partiellement le châssis central.

6. Article absorbant selon la revendication 1, dans lequel l'article comprend un deuxième objet graphique qui est imprimé partiellement sur la partie de ceinture avant et partiellement sur le châssis central.

7. Article absorbant selon la revendication 1, dans lequel l'article comprend un deuxième objet graphique qui est imprimé sur le châssis central, dans lequel un point distal du deuxième objet graphique va de 2 millimètres à 10 millimètres plus loin de l'axe latéral que de la couture de bord inférieur.
